# EUROPÄISCHE PATENTANMELDUNG

(11) **EP 3 075 334 A1**
(43) Veröffentlichungstag der Anmeldung: **05.10.2016**
(21) Anmeldenummer: 16157652.5
(22) Anmeldetag: 26.02.2016
(51) Int. Cl.: A61B 17/3209, A61M 27/00, A61M 1/00

(54) **VORRICHTUNG ZUM ENTFERNEN EINES ABSZESSINHALTES**

(30) Priorität: 02.04.2015 AT 2032015
(71) Anmelder: HOBATHERM Edelstahlkamin- und Anlagenbau GmbH, 6800 Feldkirch (AT)
(72) Erfinder: Wiesenegger, Arndt, 6840 Götzis (AT)
(74) Vertreter: Fechner, Thomas

(57) **Zusammenfassung**

Vorrichtung (1) zum Entfernen eines Abszessinhaltes (2) aus einer Abszesskammer (3) eines Hautabszesses (4), wobei die Vorrichtung (1) einen Hohlkörper (5) mit einem von einer Außenwand (6) umgebenen Hohlraum (7) und die Außenwand (6) eine Öffnung (8) zum Aufsetzen des Hohlkörpers (5) auf einen den Hautabszess (4) aufweisenden Hautbereich (9) aufweist und die Vorrichtung (1) zumindest ein Mittel (10) zur Erzeugung von Unterdruck im Hohlraum (7) und ein Stich- und/oder Schneidwerkzeug (11) zum Öffnen der Abszesskammer (3) aufweist.

## Beschreibung

Die vorliegende Erfindung betrifft eine Vorrichtung zum Entfernen eines Abszessinhaltes aus einer Abszesskammer eines Hautabszesses.

Ein Hautabszess ist eine umkapselte Ansammlung eines Abszessinhaltes, meist in Form von Eiter, Blut usw., in einer in der Haut ausgebildeten Abszesskammer. Zur Behandlung des Hautabszesses muss der Abszessinhalt meist aus der Abszesskammer entfernt werden. Dies geschieht beim Stand der Technik häufig operativ mit einem Skalpell. Ein Problem besteht darin, dass der Abszessinhalt in der Regel in der Abszesskammer unter Überdruck steht und beim Öffnen der Abszesskammer unkontrolliert in die Umgebung verspritzt wird. Dies ist unhygienisch und bedeutet sowohl für die behandelte Person oder das behandelte Tier als auch die behandelnde Person ein gewisses Infektionsrisiko.

Aufgabe der Erfindung ist es, eine Vorrichtung zum Entfernen eines Abszessinhaltes aus einer Abszesskammer eines Hautabszesses vorzuschlagen, bei der dieses Problem beseitigt ist.

Zur Lösung dieses Problems sieht die Erfindung vor, dass die Vorrichtung einen Hohlkörper mit einem von einer Außenwand umgebenen Hohlraum und die Außenwand eine Öffnung zum Aufsetzen des Hohlkörpers auf einen den Hautabszess aufweisenden Hautbereich aufweist und die Vorrichtung zumindest ein Mittel zur Erzeugung von Unterdruck im Hohlraum und ein Stich- und/oder Schneidwerkzeug zum Öffnen der Abszesskammer aufweist.

Zum Entfernen des Abszessinhaltes wird der Hohlkörper mit seiner Öffnung in der Außenwand auf den Hautbereich aufgesetzt, welcher den Hautabszess aufweist. Anschließend kann mit dem genannten Mittel ein Unterdruck im Hohlraum erzeugt werden und mit dem Stich- und/oder Schneidwerkzeug die Abszesskammer geöffnet werden. Bei dem Stich- oder Schneidwerkzeug kann es sich um eine Klinge wie z.B. bei einem Skalpell, eine Nadel, eine Spitze oder dergleichen handeln. Das Stich- und/oder Schneidwerkzeug ist dazu vorgesehen, die Hautbereiche über der Abszesskammer zu durchschneiden, aufzustechen bzw. allgemein gesprochen zu öffnen, damit der Abszessinhalt anschließend aufgrund des im Hohlraum erzeugten Unterdrucks in den Hohlkörper eingesaugt wird. Dies hat verschiedene Vorteile gegenüber dem Stand der Technik. Zum einen wird vermieden, dass der Abszessinhalt, also insbesondere der Eiter, unkontrolliert in die Umgebung spritzt. So wird der Abszessinhalt aufgrund des Unterdrucks direkt in den Hohlkörper eingesaugt, womit eine sehr hygienische Lösung geschaffen und die Infektionsgefahr sowohl für die behandelte Person bzw. gegebenenfalls auch das behandelte Tier und auch die behandelnde Person deutlich verringert ist. Zum anderen wird mit dem im Hohlraum erzeugten Unterdruck auch erreicht, dass der Abszessinhalt möglichst vollständig aus der Abszesskammer abgesaugt wird. Außerdem ist es bei Verwendung der erfindungsgemäßen Vorrichtung auf einfache Art und Weise möglich, sicher zu stellen, dass zuerst der Hohlkörper mit seiner Öffnung so auf den zu behandelnden Hautbereich aufgesetzt wird, dass die Außenwand abdichtend auf diesem Hautbereich aufsitzt, bevor der Unterdruck im Hohlraum mit dem entsprechenden Mittel der Vorrichtung erzeugt wird. Hierdurch wird sichergestellt, dass der Abszessinhalt nicht schon vor dem Aufsetzen des Hohlkörpers die Abszesskammer verlässt.

Das Stich- und/oder Schneidwerkzeug ist günstigerweise innerhalb des Hohlkörpers angeordnet. Besonders bevorzugt ist vorgesehen, dass das Stich- und/oder Schneidwerkzeug nicht aus der Öffnung im Hohlkörper nach außen hervorsteht. Das Stich- und/oder Schneidwerkzeug ist günstigerweise entweder bündig mit der durch die Öffnung aufgespannten Ebene oder relativ zu dieser Ebene ein Stück weit nach hinten in den Hohlraum versetzt angeordnet. Auch dies trägt dazu bei, dass zuerst die die Öffnung umgebende Außenwand des Hohlkörpers vollständig auf den zu behandelnden Hautbereich aufgesetzt werden kann, bevor mit dem Stich- und/oder Schneidwerkzeug die Abszesskammer geöffnet wird.

Um der behandelnden Person eine Möglichkeit zur Sichtkontrolle des Behandlungsvorgangs zu geben, ist günstigerweise vorgesehen, dass die Außenwand ganz oder bereichsweise durchsichtig ausgebildet ist. Hierdurch kann gut kontrolliert werden, dass die die Öffnung umgebende Außenwand des Hohlkörpers vollständig auf dem zu behandelnden Hautbereich mit dem Hautabszess aufgesetzt ist. Weiters kann auch kontrolliert werden, dass für den optimalen Behandlungserfolg nicht zu viel aber auch nicht zu wenig Unterdruck im Hohlraum erzeugt wird. Darüber hinaus erlaubt die zumindest bereichsweise durchsichtige Ausbildung der Außenwand auch die optische Kontrolle des Öffnens der Abszesskammer mit dem Stich- und/oder Schneidwerkzeug.

Mechanisch besonders einfache Ausgestaltungsformen der erfindungsgemäßen Vorrichtung sehen vor, dass das Stich- und/oder Schneidwerkzeug relativ zur Außenwand unbewegbar befestigt ist. Man könnte also auch von einem starr gelagerten Stich- und/oder Schneidwerkzeug sprechen. In bevorzugten Ausgestaltungsformen kann das Stich- und/oder Schneidwerkzeug dabei an der Außenwand gehalten bzw. befestigt sein, z.B. über den weiter unten noch genannten Anschlag oder einen sonstigen Steg. Bei diesen Ausgestaltungsformen kann vorgesehen sein, dass der Hautabszess nach dem Aufsetzen des Hohlkörpers auf die Haut durch die Erzeugung von Unterdruck so weit in den Hohlraum hineingesaugt wird, bis das relativ zur Außenwand unbewegbar befestigte Stich- und/oder Schneidwerkzeug in die Abszesskammer eintritt und diese öffnet, sodass der Abszessinhalt vom Unterdruck aus der Abszesskammer herausgesaugt werden kann.

Solche einfachen Ausgestaltungsformen der Erfindung bieten sich vor allem auch dann an, wenn nicht allzu komplizierte Hautabszesse zu behandeln sind oder die Behandlung auch von ungeschultem Personal oder als Selbstbehandlung durchgeführt werden soll.

In anderen Ausgestaltungsformen der Erfindung kann aber auch vorgesehen sein, dass das Stich- und/oder Schneidwerkzeug in zumindest einer Raumrichtung relativ zur Außenwand bewegbar gelagert ist. Dies bietet sich vor allem dann an, wenn es z.B. zur Behandlung etwas komplizierterer Hautabszesse möglich sein soll, das Stich- und/oder Schneidwerkzeug in definierter Art und Weise zu bewegen, um einen oder mehrere definierte Schnitt- oder Stichvorgänge durchführen zu können. Das Stich- und/oder Schneidwerkzeug kann hierzu in einer einzigen, aber auch in zwei oder drei Raumrichtungen relativ zur Außenwand bewegbar gelagert sein. Günstigerweise weisen aber auch solche Vorrichtungen eine zurückgezogene Position für das Stich- und/oder Schneidwerkzeug auf, sodass es möglich ist, die Außenwand des Hohlkörpers auf den zu behandelnden Hautbereich aufzusetzen, ohne dass das Stich- und/oder Schneidwerkzeug bei diesem Aufsetzen bereits mit dem Hautabszess in Berührung kommt. Auch bei diesen Ausgestaltungsformen der erfindungsgemäßen Vorrichtung ist günstigerweise vorgesehen, dass das Öffnen der Abszesskammer mit dem Stich- und/oder Schneidwerkzeug erst während oder nach der Erzeugung des Unterdrucks im Hohlraum erfolgt.

Um sicherzustellen, dass der Hautabszess und der ihn umgebende Hautbereich nur bis zu einer definierten Eindringtiefe mittels des Unterdrucks in den Hohlraum hineingesaugt wird, sehen bevorzugte Varianten der Erfindung vor, dass im Hohlraum, vorzugsweise an der Außenwand befestigt, zumindest ein Anschlag für den in den Hohlraum eingesaugten Hautbereich angeordnet ist. Solche Anschläge können sowohl für Ausgestaltungsformen erfindungsgemäßer Vorrichtungen mit relativ zur Außenwand unbewegbar befestigten Stich- und/oder Schneidwerkzeugen als auch für solche Ausgestaltungsformen der Erfindung vorgesehen sein, bei denen das Stich- und/oder Schneidwerkzeug in zumindest einer Raumrichtung relativ zur Außenwand bewegbar gelagert ist. Bei Ausgestaltungsformen mit feststehendem Stich- und/oder Schneidwerkzeug kann der Anschlag jedenfalls auch dafür genutzt werden, dass das Stich- und/oder Schneidwerkzeug bei Anlegen des Unterdrucks in einer definierten bzw. vorgegebenen Eindringtiefe in die Abszesskammer eindringt, wenn der Hautabszess bis zum Anschlag in den Hohlraum eingesaugt wird. In diesem Zusammenhang sehen bevorzugte Ausgestaltungsformen der Erfindung vor, dass das Stich- und/oder Schneidwerkzeug am Anschlag fixiert ist und über den Anschlag in Richtung hin zur Öffnung in der Außenwand übersteht.

Besonders einfach herstellbare und auch bedienbare Ausgestaltungsformen erfindungsgemäßer Vorrichtungen sehen vor, dass der Hohlraum zumindest bereichsweise in Form eines Kreiszylinders ausgebildet ist und die Öffnung in der Außenwand eine kreisförmige Grundfläche des Kreiszylinders bildet oder in dieser angeordnet ist. Es kann sich also z.B. um eine rohrförmige Außenwand handeln.

Für die Erzeugung des Unterdrucks im Hohlraum können verschiedenste Mittel eingesetzt werden. Diese können von Hand betätigbar aber auch maschinell betätigbar sein. Eine besonders einfach aber doch betriebssicher bedienbare Ausgestaltungsform der Erfindung sieht vor, dass das Mittel zur Erzeugung von Unterdruck im Hohlraum ein, vorzugsweise von Hand, verschiebbarer Kolben ist. Als Mittel zur Erzeugung von Unterdruck im Hohlraum kann aber auch ein Absaugdurchlass in der Außenwand vorgesehen sein, an welchen eine Absaugvorrichtung angeschlossen oder anschließbar ist. Diese Absaugvorrichtung kann Teil der Vorrichtung aber auch ein gesondertes Bauteil sein, welches direkt oder z.B. auch über einen Schlauch, ein Rohr oder dergleichen an den Absaugdurchlass angeschlossen werden kann. Beispiele für maschinell betätigbare Absaugvorrichtungen sind diverse an sich bekannte Pumpen oder dergleichen. Es kann sich aber auch um gewöhnliche, von Hand betätigbare Spritzen, Kolben-Zylindereinheiten, Bälge oder dergleichen handeln.

Besonders bevorzugte Ausgestaltungsformen der Erfindung sehen vor, dass die Öffnung in der Außenwand von einer flexiblen Membran abgedeckt ist. Mit dieser flexiblen Membran kann sichergestellt werden, dass der aus der Abszesskammer abgesaugte Abszessinhalt auch nach Ende der Behandlung im Hohlraum der Vorrichtung verbleibt. Es kann vorgesehen sein, dass die flexible Membran vor der Verwendung der Vorrichtung die Öffnung der Außenwand vollständig abschließt und erst beim Aufbau des Unterdrucks im Hohlraum von dem Stich- und/oder Schneidwerkzeug durchstochen wird. Besonders bevorzugt besteht die flexible Membran aus einem elastischen Material wie z.B. einem Elastomer. Als Beispiel kann eine Silikonmembran mit einer Dicke von z.B. 2mm genannt werden. Insbesondere bei solchen elastischen flexiblen Membranen kann aber auch vorgesehen sein, dass diese Membran eine vorgefertigte Schnittfuge zum Hindurchtreten des Stich- und/oder Schneidwerkzeugs aufweist. Diese Schnittfuge kann dann vor Anlegen des Unterdrucks im Hohlraum und nach Abbau des Unterdrucks im Hohlraum z.B. durch elastische Rückstellkräfte des elastischen Materials der Membran geschlossen sein und sich nur während des Behandlungsvorgangs durch den Unterdruck im Hohlraum öffnen, sodass das Stich- und/oder Schneidwerkzeug durch die Schnittfuge hindurchdringen kann und auch der Abszessinhalt aus der Abszesskammer durch die Schnittfuge hindurch in den Hohlraum eingesaugt werden kann.

Bei erfindungsgemäßen Vorrichtungen kann es sich sowohl um für die Einmalverwendung vorgesehene Einwegprodukte handeln, welche anschließend nach der Behandlung eines Hautabszesses weggeworfen werden. Es kann sich aber auch um Mehrwegprodukte handeln, welche mehrfach zur Behandlung von Hautabszessen eingesetzt werden. Unabhängig davon, ob es sich um Einweg- oder Mehrwegprodukte handelt, ist bevorzugt vorgesehen, dass die Vorrichtung vor der Verwendung sterilisiert ist. Besonders bevorzugte Ausgestaltungsformen sehen vor, dass die Vorrichtung sterilisiert verpackt, z.B. in eine entsprechende Folie oder einen entsprechenden Beutel eingeschweißt ist oder dergleichen. Dies kann sowohl bei der Ausführung als Einwegprodukt als auch bei der Ausführung als Mehrwegprodukt vorgesehen sein.

Insbesondere bei Ausgestaltungsformen mit, die Öffnung der Außenwand verschließenden, flexiblen Membranen kann vorgesehen sein, dass sich im Hohlraum der noch nicht verwendeten Vorrichtung ein Desinfektionsmittel oder dergleichen befindet. Dies kann natürlich auch direkt auf dem Stich- und/oder Schneidwerkzeug aufgetragen sein. In beiden Ausgestaltungsformen sorgt das Desinfektionsmittel dann automatisch bei der Behandlung des Hautabszesses mit der erfindungsgemäßen Vorrichtung auch für die notwendige Desinfektion. Weitere Merkmale und Einzelheiten bevorzugter Ausgestaltungsformen der Erfindung werden beispielhaft anhand der hier gezeigten schematisierten Ausführungsbeispiele erläutert. Es zeigen:
Fig. 1 und 2 Längsschnitte in um 90° gedrehten Richtungen durch ein erstes schematisiert dargestelltes Ausführungsbeispiel der Erfindung;
Fig. 3 und 4 zu den Fig. 1 und 2 entsprechende Längsschnitte bei der Behandlung des Hautabszesses mit diesem ersten Ausführungsbeispiel der Erfindung und
Fig. 5 und 6 Längsschnitte durch weitere Ausgestaltungsformen der Erfindung.

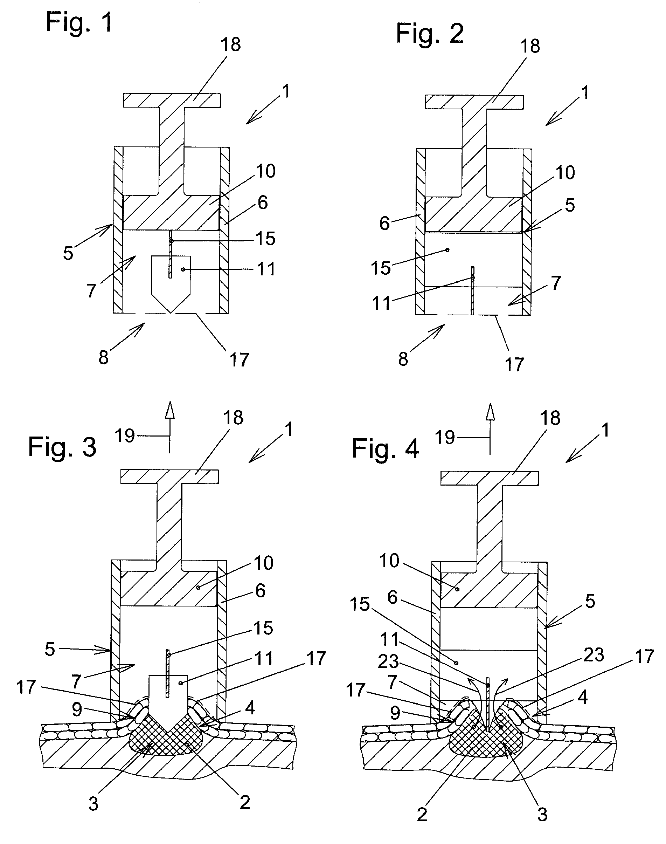

Die Fig. 1 und 2 zeigen schematisierte Längsschnitte durch ein Ausführungsbeispiel einer erfindungsgemäßen Vorrichtung 1. Die beiden Schnitte sind bezüglich einer gedachten Längsachse durch die Vorrichtung 1 um 90° gegeneinander verdreht. Der Hohlkörper 5 der Vorrichtung 1 ist in diesem Ausführungsbeispiel rohrförmig ausgebildet. Die den Hohlraum 7 ummantelnde Außenwand 6 des Hohlkörpers 5 ist als Mantelwand eines Kreiszylinders ausgeführt. Die auf den den Hautabszess 4 aufweisenden Hautbereich 9 aufzusetzende Öffnung 8 des Hohlkörpers 5 ist in diesem Ausführungsbeispiel kreisförmig ausgebildet. Sie kann von einer hier strichliert dargestellten optionalen Membran 17 verschlossen sein. Auf die Membran 17 kann aber auch verzichtet werden. Die Membran 17 kann, soweit sie vorhanden ist, vor Verwendung der Vorrichtung 1 vollständig geschlossen sein und die Öffnung 8 vollständig abdichten. In der Membran 17 kann aber auch bereits eine vorgefertigte Schnittfuge für das Stich- und/oder Schneidwerkzeug 11 vorhanden sein, welche sich vorzugsweise aber erst dann öffnet, wenn Unterdruck im Hohlraum 7 angelegt wird.

Die Außenwand 6 ist zumindest bereichsweise durchsichtig ausgebildet. Ist sie nicht vollständig durchsichtig ausgebildet, so sollten die Sichtfenster so angeordnet sein, dass man bei der Behandlung des Hautabszesses 4 sowohl den Hautabszess 4 als auch das Stich- und/oder Schneidwerkzeug 11 von außen sehen kann. Im gezeigten Ausführungsbeispiel ist das Stich- und/oder Schneidwerkzeug 11 als feststehende Klinge ausgeführt, welche relativ zur Außenwand 6 unbewegbar befestigt ist. Als Mittel 10 zur Erzeugung von Unterdruck im Hohlraum 7 ist bei der ersten gezeigten Variante der Erfindung ein verschiebbarer Kolben vorgesehen. Dieser ist gegen die Außenwand 6 des Hohlkörpers 5 abgedichtet, sodass durch Ziehen an diesem Mittel 10 bzw. Kolben in Zugrichtung 19 Unterdruck im Hohlraum 7 erzeugt werden kann, wenn die Außenwand 6 mit der Öffnung 8 auf den zu behandelnden Hautbereich 9 entsprechend aufgesetzt ist. Im Hohlraum 7 befindet sich aber nicht nur das Stich- und/oder Schneidwerkzeug 11 sondern auch ein Anschlag 15, durch den vorgegeben ist, wie weit der Hautbereich 9 in den Hohlraum 7 durch entsprechendes Anlegen von Unterdruck bzw. Ziehen am Kolben 10 eingesaugt werden kann. Am Kolben 10 befindet sich für die händische Betätigung in diesem Ausführungsbeispiel ein Handgriff 18. Im ersten Ausführungsbeispiel erfüllt der Anschlag 15 eine Doppelfunktion. Zum einen begrenzt er, wie weit der Hautbereich 9 in den Hohlraum 7 eingesaugt werden kann. Zum anderen ist an ihm aber auch das Stich- und/oder Schneidwerkzeug 11 fixiert. Der Anschlag 15 ist in diesem ersten Ausführungsbeispiel als ein, den Hohlraum 7 querender und von beiden Seiten an der Außenwand 6 befestigter Steg ausgebildet. Natürlich kann ein solcher Anschlag 15 auch anders ausgebildet und/oder am oder im Hohlkörper 5 befestigt sein. Es ist auch nicht zwingend notwendig, dass der Anschlag 15 gleichzeitig der Träger des Stich- und/oder Schneidwerkzeugs 11 ist.

Eine gegebenenfalls vorhandene sterile Verpackung der Vorrichtung 1 kann z. B. in Form eines Beutels, in den die Vorrichtung 1 eingeschweißt ist, ausgeführt sein. Sterile Verpackungen von medizinischen Vorrichtungen sind an sich bekannt und müssen hier nicht weiter erläutert werden.

Mit der Vorrichtung 1 dieses ersten Ausführungsbeispiels kann ein Hautabszess 4 wie folgt behandelt werden:
Zunächst wird die Außenwand 6 des Hohlkörpers 5 so auf den gegebenenfalls vorab desinfizierten, den Hautabszess 4 aufweisenden, Hautbereich 9 aufgesetzt, dass der Hautabszess 4 sich in der Öffnung 8 des Hohlkörpers 5 befindet. Ist die Membran 17 vorhanden, so liegt sie dann entsprechend auf dem Hautbereich 9 und damit auch auf dem Hautabszess 4 auf. Die Außenwand 6 wird so gegen die Haut gedrückt, dass das notwendige Maß an Abdichtung zwischen Außenwand 6 und Hautbereich 9 erreicht wird. Nun wird durch Ziehen in Zugrichtung 19 am Handgriff 18 der Kolben 10 nach oben gezogen, um so den gewünschten Unterdruck im Hohlraum 7 zu erzeugen. Durch den Unterdruck wird der Hautabszess 4 bzw. der Hautbereich 9 in den Hohlraum 7 eingesaugt, sodass es zu der in den Fig. 3 und 4 gut sichtbaren Aufwölbung kommt. Ist die Membran 17 vorhanden, so wird sie ebenfalls entsprechend mit in den Hohlraum 7 eingesaugt und aufgewölbt. Durch dieses Aufwölben des Hautbereichs 9 bzw. des Hautabszesses 4 und gegebenenfalls auch der Membran 17 dringt das Stich- und/oder Schneidewerkzeug 11, gegebenenfalls durch die Membran 17 hindurch, in die Abszesskammer 3 ein, wodurch diese geöffnet wird. Der Unterdruck saugt dann den Abszessinhalt 2 aus der Abszesskammer 4 in den Hohlraum 7. Das Eindringen des Stich- und/oder Schneidwerkzeugs 11 in die Abszesskammer 3 ist in den Fig. 3 und 4 schematisiert dargestellt. Die Pfeile 23 zeigen symbolisch das Aussaugen der geöffneten Abszesskammer 3 und damit den Übertritt des Abszessinhaltes 2, also insbesondere des Eiters, in den Hohlraum 7. Der Anschlag 15 gibt dabei vor, wie weit der Hautabszess 4 in den Hohlraum 7 hineingesaugt werden kann. Hierdurch ist automatisch auch die Eindringtiefe des hier relativ zur Außenwand 6 fix befestigten Stich- und/oder Schneidwerkzeuges 11 in die Abszesskammer 3 vorgegeben. Durch den Unterdruck einerseits und die Hautspannung andererseits wird die Schnittstelle, also der Bereich in dem das Stich- und/oder das Schneidwerkzeug 11 in die Abszesskammer 3 eingedrungen ist, auseinandergezogen und dadurch eine große Hautöffnung geschaffen, wodurch der Abszessinhalt 2 besonders vollständig aus der Abszesskammer 3 entfernt werden kann. Dies kann, falls notwendig, auch dadurch noch unterstützt werden, dass man außerhalb der Vorrichtung 1, die den Hautbereich 9 umgebende Haut noch zusätzlich z. B. mit den Fingern zusammendrückt.

Ist der Abszessinhalt 2 in der gewünschten Art und Weise aus der Abszesskammer 3 abgesaugt worden, so kann anschließend der Unterdruck durch Wegziehen der Vorrichtung 1 von der Haut oder durch Rückführen des Kolbens 11 wieder ausgeglichen werden. Hierdurch geht die Spannung und Wölbung der Haut zurück und die Schnittöffnung in der Abszesskammer 3 wird verkleinert. Ist die flexible Membran 17 vorhanden, so geht diese wieder in ihre Ausgangsstellung zurück und verschließt auch die Durchtrittsstelle durch die das Stich- und/oder Schneidwerkzeug 11 hindurchgedrungen ist. Ist die Membran 17 vorhanden, so sorgt sie dann dafür, dass der aus der Abszesskammer 3 abgesaugte Abszessinhalt 2 im Hohlraum 7 der Vorrichtung 1 verbleibt und nicht nach außen dringen kann. Insbesondere wenn die Membran 17 nicht vorhanden ist, kann der Hautbereich 9 nach Entfernen der Vorrichtung 1 aber auch noch einfach und schnell gereinigt bzw. desinfiziert werden.

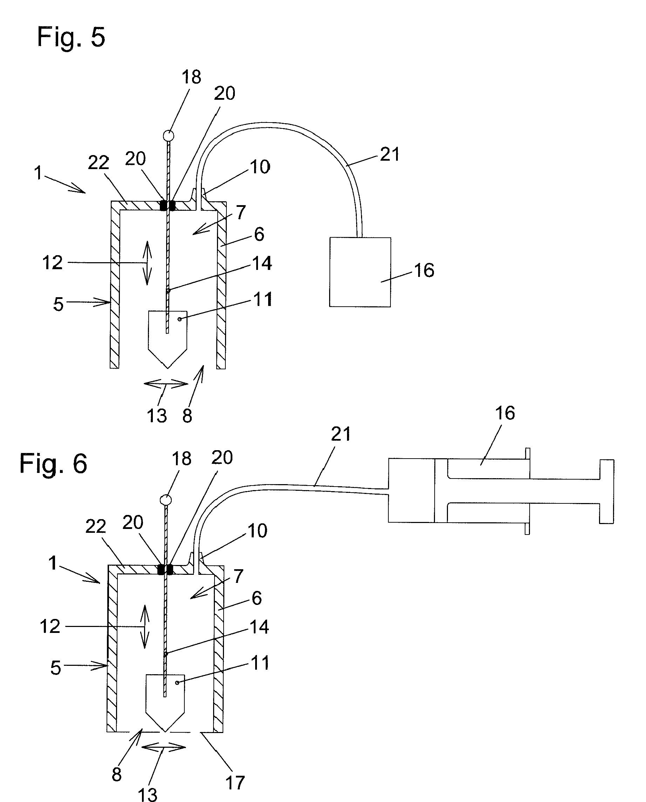

Fig. 5 zeigt, ebenfalls in einem schematisierten Längsschnitt, ein zweites Ausführungsbeispiel der Erfindung. Hier ist die Außenwand 6 des Hohlkörpers 5 der Vorrichtung 1 zwar auch bereichsweise zylindermantelförmig ausgeführt. Das der Öffnung 8 gegenüberliegende Ende des Hohlraums 7 ist aber durch den Deckel 22 verschlossen. Eine den Handgriff 18 mit dem Stich- und/oder Schneidwerkzeug 11 verbindende Stange 14 ist durch den Deckel 22 hindurchgeführt, wobei diese Hindurchführung mit der Dichtung 20 druckfest abgedichtet ist. Hierdurch kann durch Betätigen des Handgriffs 18 das Stich- und/oder Schneidwerkzeug 11 in diesem Ausführungsbeispiel zum Öffnen der Abszesskammer 3 relativ zur Außenwand 6 bewegt werden. Es kann sich, wenn es nur um ein Aufstechen der Abszesskammer 3 geht, um eine rein lineare Stichbewegung parallel zur Raumrichtung 12 handeln. In anderen Ausführungsbeispielen kann aber sowohl anstelle der Bewegung in Raumrichtung 12 als auch zusätzlich eine Bewegung in zumindest einer anderen Raumrichtung 13 möglich sein, z. B. um Schnittbewegungen zum Öffnen der Abszesskammer 3 durchzuführen. Auf diese Art und Weise kann das Stich- und/oder Schneidwerkzeug 11 auch in zwei oder drei Raumrichtungen 12, 13 bewegbar sein. Bevorzugt gibt es aber jedenfalls eine zurückgezogene Position des Stich- und/oder Schneidwerkzeuges 11, in der die Außenwand 6 auf den zu behandelnden Hautbereich 9 aufgesetzt werden kann, ohne dass es dabei gleich zum Öffnen der Abszesskammer 3 kommt.

Im Ausführungsbeispiel gemäß Fig. 5 weist die dort gezeigte Vorrichtung 1 als Mittel 10 zur Erzeugung von Unterdruck im Hohlraum 7 einen Absaugdurchlass in der Außenwand 6 auf. An diesen kann, hier im Beispiel über einen Schlauch 21, eine Absaugvorrichtung 16 angeschlossen werden, mit der der Unterdruck im Hohlraum 7 erzeugt werden kann. Diese Absaugvorrichtung 16 kann fixer Bestandteil der Vorrichtung 1 aber auch ein z. B. mehrfach verwendbares separates Bauteil, z. B. in Form einer Pumpe o. dgl. sein. Über den Schlauch 21 kann auch der Abszessinhalt 2 aus dem Hohlraum 7 abgesaugt werden, falls dies gewünscht ist.

Fig. 6 zeigt in einem entsprechenden Längsschnitt nun noch ein drittes Ausführungsbeispiel der Erfindung, welches eine Abwandlungsform des zweiten Ausführungsbeispiel gemäß Fig. 5 ist. Der eine Unterschied besteht darin, dass hier im Gegensatz zu Fig. 5 beispielhaft wiederum die bereits besprochene Membran 17 vorhanden ist. Der andere Unterschied besteht in der Ausgestaltungsform der Absaugvorrichtung 16. In der Variante gemäß Fig. 6 handelt es sich hierbei um eine gewöhnliche, von Hand betätigbare Spritze mit der durch Ziehen am Kolben der Spritze über den Schlauch 21 Unterdruck im Hohlraum 7 erzeugt werden kann.

Alle hier gezeigten Ausführungsbeispiele der Erfindung haben den Vorteil, dass beim Öffnen der Abszesskammer 3 der Abszessinhalt 2 nicht unkontrolliert in die Umgebung spritzen kann. Hierdurch ist die Infektionsgefahr sowohl für die behandelte Person bzw. das behandelte Tier als auch für die behandelnde Person z. B. einen Arzt oder eine Krankenschwester, als auch für die sonstige Umgebung deutlich verringert. Durch den erzeugten Unterdruck haben erfindungsgemäße Vorrichtungen 1 auch den weiteren Vorteil, dass der Abszessinhalt 2 mehr oder weniger vollständig aus der Abszesskammer 3 entfernt wird. Insbesondere bei Ausgestaltungsformen mit Membran 17 kann der Abszessinhalt 2 im Hohlraum 7 gesammelt und verschlossen werden, z. B. um so zur weiteren Untersuchung an ein Labor versendet werden zu können. Ein weiterer Vorteil erfindungsgemäßer Vorrichtungen 1 liegt in der schnellen und unkomplizierten Behandlung des Hautabszesses 4. Erfindungsgemäße Vorrichtungen 1 können nicht nur von Ärzten sondern auch von Laien bedient werden und sind zumindest bei kleineren Hautinfektionen auch für die Selbstbehandlung des Patienten geeignet. Wie eingangs bereits erläutert, kann es sich um Einweg- aber auch Mehrwegvarianten handeln. Eine sterile Verpackung ist bevorzugt vorgesehen. Erfindungsgemäße Vorrichtungen 1 können in unterschiedlichen Größen und mit unterschiedlichen Einschnitttiefen ausgeführt werden. Erfindungsgemäße Vorrichtungen 1 sind darüber hinaus sowohl in der Human- als auch in der Veterinärmedizin einsetzbar.

### Legende zu den Hinweisziffern:

- 1: Vorrichtung
- 2: Abszessinhalt
- 3: Abszesskammer
- 4: Hautabszess
- 5: Hohlkörper
- 6: Außenwand
- 7: Hohlraum
- 8: Öffnung
- 9: Hautbereich
- 10: Mittel zur Erzeugung von Unterdruck
- 11: Stich- und/oder Schneidwerkzeug
- 12: Raumrichtung
- 13: Raumrichtung
- 14: Stange
- 15: Anschlag
- 16: Absaugvorrichtung
- 17: Membran
- 18: Handgriff
- 19: Zugrichtung
- 20: Dichtung
- 21: Schlauch
- 22: Deckel
- 23: Pfeil

## Patentansprüche

1. Vorrichtung (1) zum Entfernen eines Abszessinhaltes (2) aus einer Abszesskammer (3) eines Hautabszesses (4), **dadurch gekennzeichnet, dass** die Vorrichtung (1) einen Hohlkörper (5) mit einem von einer Außenwand (6) umgebenen Hohlraum (7) und die Außenwand (6) eine Öffnung (8) zum Aufsetzen des Hohlkörpers (5) auf einen den Hautabszess (4) aufweisenden Hautbereich (9) aufweist und die Vorrichtung (1) zumindest ein Mittel (10) zur Erzeugung von Unterdruck im Hohlraum (7) und ein Stich- und/oder Schneidwerkzeug (11) zum Öffnen der Abszesskammer (3) aufweist.

2. Vorrichtung (1) nach Anspruch 1, **dadurch gekennzeichnet, dass** das Stich- und/oder Schneidwerkzeug (11) innerhalb des Hohlraums (7) angeordnet ist.

3. Vorrichtung (1) nach Anspruch 1 oder 2, **dadurch gekennzeichnet, dass** die Außenwand (6) ganz oder bereichsweise durchsichtig ausgebildet ist.

4. Vorrichtung (1) nach einem der Ansprüche 1 bis 3, **dadurch gekennzeichnet, dass** das Stich- und/oder Schneidwerkzeug (11) relativ zur Außenwand (6) unbewegbar befestigt ist.

5. Vorrichtung (1) nach einem der Ansprüche 1 bis 3, **dadurch gekennzeichnet, dass** das Stich- und/oder Schneidwerkzeug (11) in zumindest einer Raumrichtung (12, 13) relativ zur Außenwand (6) bewegbar gelagert ist.

6. Vorrichtung (1) nach einem der Ansprüche 1 bis 5, **dadurch gekennzeichnet, dass** im Hohlraum (7), vorzugsweise an der Außenwand (6) befestigt, zumindest ein Anschlag (15) für den in den Hohlraum (7) eingesaugten Hautbereich (9) angeordnet ist.

7. Vorrichtung (1) nach Anspruch 6, **dadurch gekennzeichnet, dass** das Stich- und/oder Schneidwerkzeug (11) am Anschlag (15) fixiert ist und über den Anschlag (15) in Richtung hin zur Öffnung (8) in der Außenwand (6) übersteht.

8. Vorrichtung (1) nach einem der Ansprüche 1 bis 7, **dadurch gekennzeichnet, dass** der Hohlraum (7) zumindest bereichsweise in Form eines Kreiszylinders ausgebildet ist und die Öffnung (8) in der Außenwand (6) eine kreisförmige Grundfläche des Kreiszylinders bildet oder in dieser angeordnet ist.

9. Vorrichtung (1) nach einem der Ansprüche 1 bis 8, **dadurch gekennzeichnet, dass** das Mittel (10) zur Erzeugung von Unterdruck im Hohlraum (7) ein, vorzugsweise von Hand, verschiebbarer Kolben ist und/oder dass das Mittel (10) zur Erzeugung von Unterdruck im Hohlraum (7) ein Absaugdurchlass in der Außenwand (6) ist, an welchen eine Absaugvorrichtung (16) angeschlossen oder anschließbar ist.

10. Vorrichtung (1) nach einem der Ansprüche 1 bis 8, **dadurch gekennzeichnet, dass** die Öffnung (8) in der Außenwand (6) von einer flexiblen Membran (17) abgedeckt ist.
